# EUROPEAN PATENT SPECIFICATION

(11) **EP 2 886 341 B1**
(45) Date of publication and mention of the grant of the patent: **07.02.2018**
(21) Application number: 14197805.6
(22) Date of filing: 12.12.2014
(51) Int. Cl.: B32B 37/20, B32B 3/18, A61F 13/49

(54) **Stretch laminate with curved elastic and corresponding method**
Stretch-Laminat mit gebogener Elastik und korrespondierendes Verfahren
Laminé extensible avec élastique courbe et méthode correspondante

(30) Priority: 12.12.2013 US 201361915206 P
(43) Date of publication of application: 24.06.2015
(73) Proprietor: Curt G. Joa, Inc., Sheboygan Falls, Wisconsin 53085 (US)
(72) Inventor: Schwartz, Christopher A., Sheboygan, WI Wisconsin 53085 (US); Andrews, Robert E., Sheboygan, WI Wisconsin 53083 (US)
(74) Representative: Potter Clarkson LLP

(56) References cited:
- US-A1- 2006 121 252
- US-A1- 2006 292 328

## Description

### Background of the Invention

This invention relates to formation of stretch laminates containing curved elastic. Disposable garments, and more particularly, a pants-type diaper, are equipped with elastic strands effectively encircling the leg-holes without traversing the crotch region. A method for producing such diapers is disclosed.

Disposable diapers of the children's training pant type, or of the adult incontinence type, are typically equipped with elastic strands, which encircle the leg-holes. These strands of elastic are typically captured with adhesive between two layers of non-woven materials. Various methods are used to position these elastic strands so that they produce the desired encircling effect.

In one method of manufacture, the diapers are produced in an orientation whereby product flow is in the form of a single continuous web and the direction of travel is at a right angle with respect to what would be described as the crotch line of the diaper, i.e., the normal direction of product flow is parallel to the waist as opposed to parallel to the crotch.

One method of creating the desired effect of encircling the leg holes of the pant with elastics is to interleave two swaths of elastic strands, each curving across the face of the traveling web, encircling about one half of the leg-hole areas and crossing the path of the other. As a pair, they create a boundary around each leg-hole cutout, which resembles a circle or ellipse. In practice, however, the lateral excursions of the elastic lay-down device are speed-limited. As the traveling web is moving at some speed in one direction, and as the elastic lay-down device has speed and acceleration limits in the cross-direction, there is a limit to the steepness of the oblique angle which it is possible to form between the two. The result of this limitation is usually seen in the form of apparent incompleteness in the formation of the leg-hole-encircling pattern, particularly at the crotch line, where the two swaths cross each other.

From the point on the web at which one leg-hole pattern has been completed to the point at which the next can be begun, the elastic laydown device must reposition itself to a favorable starting point. This period of repositioning occurs as the crotch region passes the laydown device. As a result, the elastic strands must also cross this region of the product, at which they may or may not be attached by means of adhesives to the carrier webs. Various means are used to control or limit the positional relationships of the elastic strands in this region. The two sets of strands may cross over each other, creating an "X" pattern, or, they may loop back over to their respective sides, creating an "O" at the center of the crotch region. Alternatively, they may be mechanically stopped and prevented from crossing each other, creating two sets of generally parallel lines at the crotch The lay-down pattern used at the crotch will determine the final appearance of the product in this area.

The shirring effect created by elastic strands when laminated with any flexible fabric is well known. However, to have this shirring effect applied to the crotch of a pant-type garment can be undesirable. The elastics create a contractile force, which tends to distort the garment at this location, thereby reducing the garment's aesthetic appeal, effectiveness and comfort. Thus various methods of reducing or eliminating the effects of the elastic tension normally occurring at the crotch have been attempted. These methods include the elimination of the adhesive bond between the strands and the liner materials described in U.S. Patent 5,745,922 as "unsecured space" as well as various methods of cutting the strands to eliminate their effects.

As mentioned, one method of eliminating the undesired effects of the elastic strands which cross the crotch region is to sever them. This method is described in U. S. Patent 5,660,657. Unfortunately, such severing usually requires the introduction of a transversely extending cut, which can result in a loss of web tension in the severed part of the carrier web. This also creates an undesirable opening in the diaper backsheet. A proposed solution for this problem is taught in U. S. Patent 5,707,470, wherein an ultrasonic device is used to sever the elastic members, while the carrier webs which encapsulate the elastics are left intact. See, also, U. S. Patent 5,643,396. Another problem associated with such severing lies in the tendency of the unsecured severed ends of elastic to retract to some point beyond the limits of any adhesive pattern. Thus, the elastic strands are not controlled or anchored near the ends of the adhesion pattern and may snap back to further into the adhesive pattern. This results in an incomplete elastic pattern and poor product characteristics.

One method of compensating for the incompleteness of the encircling pattern entails insertion of an additional set of elastic strips, running parallel to the crotch line and transverse to the web path. See U. S. Patents 5,634,917 and 5,660,657. Typical products of this type are provided with an outer laminate, which is formed of an inner liner material and an outer backsheet material, between which the leg-hole elastics are disposed.

Often, leg elastics or other types of continuous ribbons are applied to running webs in a sinusoidal pattern by a roll-fed web process. Roll-fed web processes typically use a constant infeed rate, which in the case of a sinusoidal ribbon application, can result in necking, or undesirable narrowing of the ribbon toward the inner and outer portions of the sine curve in the cross-machine direction. This is because the infeed rate of the ribbon web does not match with the velocity of the substrate it is being laid upon in the machine direction. Instead, the ribbon material is stretched somewhat at the extremities of the sine curve.

Roll-fed web processes typically use splicers and accumulators to assist in providing continuous webs during web processing operations. A first web is fed from a supply wheel (the expiring roll) into the manufacturing process. As the material from the expiring roll is depleted, it is necessary to splice the leading edge of a second web from a standby roll to the first web on the expiring roll in a manner that will not cause interruption of the web supply to a web consuming or utilizing device.

In a splicing system, a web accumulation dancer system may be employed, in which an accumulator collects a substantial length of the first web. By using an accumulator, the material being fed into the process can continue, yet the trailing end of the material can be stopped or slowed for a short time interval so that it can be spliced to leading edge of the new supply roll. The leading portion of the expiring roll remains supplied continuously to the web-utilizing device. The accumulator continues to feed the web utilization process while the expiring roll is stopped and the new web on a standby roll can be spliced to the end of the expiring roll.

In this manner, the device has a constant web supply being paid out from the accumulator, while the stopped web material in the accumulator can be spliced to the standby roll. Examples of web accumulators include that disclosed in U.S. Patent Application Serial No. 11/110,616, which is commonly owned by the assignee of the present application.

Examples of curved elastic application are disclosed in U.S. Patent No. 6,482,278.

Finally, a laminate comprising elastic strips, with elastic and non-elastic regions, is disclosed in US patent application 2006/0292328 A1.

### Summary of the Invention

According to the present invention there is provided a method of forming a laminate according to claim 1 and a laminate according to claim 4.

An adhesive zone and an ultrasonic zone are used to join elastics to nonwoven layers to from front and rear portions of a diaper as a laminate.

An adhesive zone is supplied to join elastic strands laid down in a curved fashion to a nonwoven layer. An ultrasonic zone is supplied to bond an elastomeric layer with a second nonwoven layer. This zone is preferably adhesive free. The elastic strands and first nonwoven layer are next bonded to the elastomeric layer and second nonwoven layer.

A number of product iterations are possible such as curved elastic on the front panel as well as the rear panel. In this case the front panel could also present a leg curved or contoured shape.

A two step procedure is disclosed in which a bi-laminate of nonwoven material and an elastomeric layer are made independently, and then brought together with a bi-laminate of nonwoven that has adhesive added for curved elastic strands. When the bi-laminate of nonwoven material and an elastomeric layer is brought together with the bi-laminate of nonwoven that has adhesive added for curved elastic strands, the curved elastic is preferably introduced just prior to the combining point for near instant capture of the curved elastic.

### Brief Description of the Drawings

Fig. 1 is a side view of a prior art laminate in which a nonwoven layer covers elastic strands laid atop a second nonwoven layer, laid atop an elastomeric layer coupled to a bottom nonwoven layer;
Fig. 2 is a side schematic view of a laminate forming mechanism of the present invention in which a top nonwoven layer is applied with adhesive, and elastic strands are introduced to the top nonwoven layer prior to a combining point, at which a previously ultrasonically bonded elastomeric layer has been bonded with a bottom nonwoven layer;
Fig. 3 is a plan view of a diaper produced according to the methods of the present invention;
Fig. 4 is a cross sectional view of the diaper shown in Fig. 3.

### Description of the Preferred Embodiment

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention, which is defined by the claims.

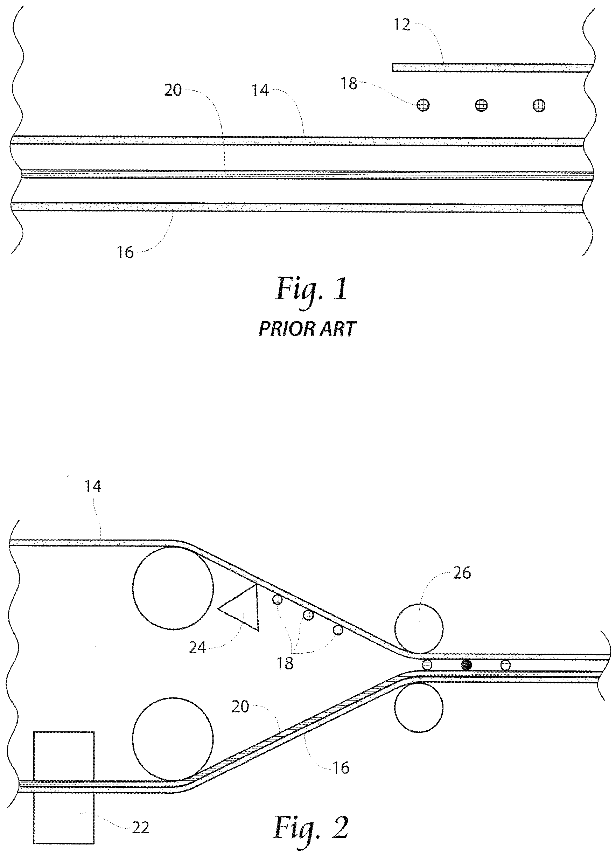

Referring now to Fig. 1, a side view of a prior art laminate in which a nonwoven layer 12 covers elastic strands 18 (which can be laid down in curved fashion according to known methods) laid atop a second nonwoven layer 14, laid atop an elastomeric layer 20 coupled to a bottom nonwoven layer 16 is shown. Adhesives are generally used to create the bonds in this laminate between layers.

Referring now to Fig. 2, a side schematic view of a laminate forming mechanism of the present invention is shown. A top nonwoven layer 14 is applied with adhesive by adhesive applicator 24. Elastic strands 18 are introduced to the top nonwoven layer prior to a combining point 26. Preferably, the curved elastic 18 is introduced to top nonwoven 14 just prior the combining point 26 for near instant capture of the curved elastic 18. An ultrasonic bonding unit 22 bonds elastomeric layer 20 with a bottom nonwoven layer 16, and this bi-laminate is fed to bonding unit or combining point 26 to receive the previously produced curved elastic 18 combination with top nonwoven 14. This laminate is fed downstream for further processing as desired.

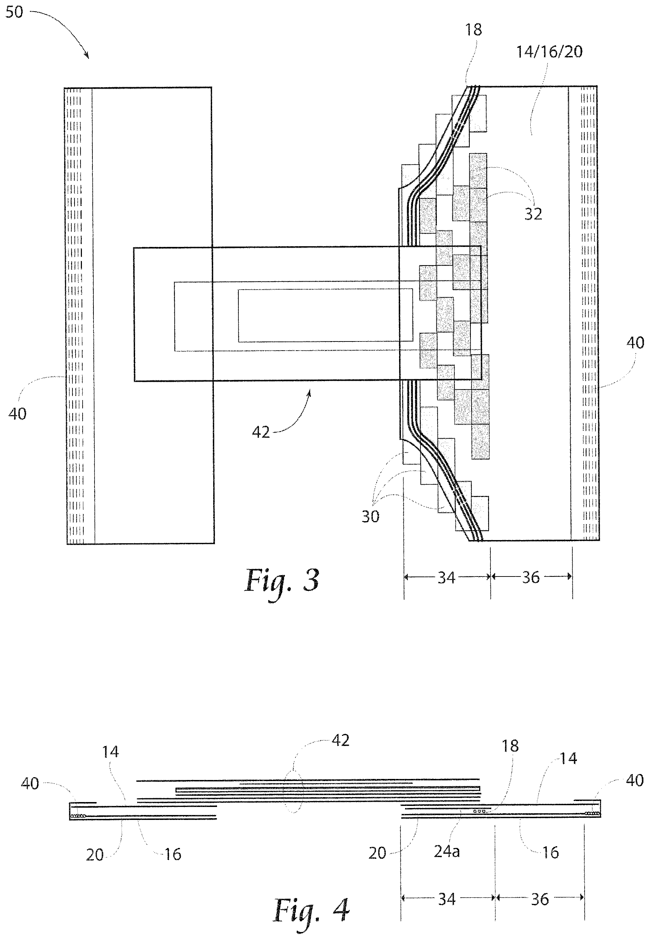

The resulting laminate of this configuration can be used in a diaper product 50 as shown Fig. 3. As shown in Fig. 3, the diaper has a front panel (shown at left) comprised of a stretch laminate with a top nonwoven layer 14, bottom nonwoven layer 16, and both an elastomeric layer 20 and waist elastics 40 supplied. The bottom nonwoven 16 can be folded over at the left side in order to capture the waist elastics 40 and the top nonwoven 14.

A rear panel (shown at right) has two distinct bonding zones, an adhesive zone 34 and an ultrasonic zone 36. The adhesive zone is defined by the adhesive applicator 24 (Fig. 2) as where either zoned or continuous adhesive has been laid to top nonwoven layer 14. A portion of the adhesive 24a (seen in cross section, Fig. 4) laid down in adhesive pattern portions 30 presents a target area for elastic strands 18. A second adhesive pattern 32 presents a portion of adhesive zone 34 targeted to couple the elastomeric/nonwoven bilaminate 20/16 to the top laminate 14.

In ultrasonic zone 36, it is preferable that no adhesives are present, and elastomeric/nonwoven bilaminate 20/16 and top laminate 14 are coupled ultrasonically.

As with the front panel, bottom nonwoven 16 can be folded over at the right side in order to capture waist elastics 40 and the top nonwoven 14.

Still referring to Fig. 3, core components 42 are used to bridge the front and rear panels. Core components 42 can comprise acquisition layers, absorbent core material including superabsorbent polymer (SAP) and cellulosic fluff absorbent material, and nonwoven materials as preferred by the manufacturer. Core components are coupled to the front and rear panels, for instance adhesively or ultrasonically. The assembled components are shown in Fig. 4 in cross section.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. A method of forming a laminate comprising:
forming independently a first laminate comprising a base layer (16) coupled to an elastomeric layer (20);
forming independently a second laminate comprising a top layer (14) coupled to a series of curved elastic strands (18) by an adhesive (24a); and
coupling said first laminate to said second laminate ultrasonically.

2. A method according to claim 1, said base layer (16) coupled to said elastomeric layer (20) ultrasonically.

3. A method according to claim 1, wherein the top layer (14) is a non-woven layer and the adhesive is applied to the top layer.

4. A laminate formed by the method according to claim 1, said laminate comprising an adhesive zone (34) representing a location where said top layer (14) is coupled to said series of curved elastic strands (18), and an ultrasonic zone (36) representing a location where said first laminate is coupled ultrasonically to said second laminate.

5. A laminate according to claim 4, said ultrasonic zone (36) containing no adhesives.

6. A laminate according to claim 4, said adhesive zone (34) comprising a first adhesive pattern (30) for coupling the curved elastic strands (18) to the top layer (14) and a second adhesive pattern (32) for coupling the top layer (14) to the first laminate.

## Patentansprüche

1. Verfahren zum Ausbilden eines Laminats, Folgendes umfassend:
unabhängiges Ausbilden eines ersten Laminats, das eine Basisschicht (16), gekoppelt an eine elastomere Schicht (20), umfasst;
unabhängiges Ausbilden eines zweiten Laminats, das eine Deckschicht (14), durch einen Klebstoff (24a) an eine Reihe von gebogenen, elastischen Strängen (18) gekoppelt, umfasst; und
Koppeln des ersten Laminats an das zweite Laminat mittels Ultraschall.

2. Verfahren nach Anspruch 1, wobei die Basisschicht (16) mittels Ultraschall an die elastomere Schicht (20) gekoppelt wird.

3. Verfahren nach Anspruch 1, wobei die Deckschicht (14) eine Vliesschicht ist und der Klebstoff auf die Deckschicht aufgetragen wird.

4. Laminat, ausgebildet durch das Verfahren nach Anspruch 1, wobei das Laminat Folgendes umfasst: einen Klebstoffbereich (34), der eine Stelle darstellt, an welcher die Deckschicht (14) an die Reihe von gebogenen, elastischen Strängen (18) gekoppelt ist, und einen Ultraschallbereich (36), der eine Stelle darstellt, an welcher das erste Laminat mittels Ultraschall an das zweite Laminat gekoppelt ist.

5. Laminat nach Anspruch 4, wobei der Ultraschallbereich (36) keine Klebstoffe enthält.

6. Laminat nach Anspruch 4, wobei der Klebstoffbereich (34) ein erstes Klebstoffmuster (30) zum Koppeln der gebogenen, elastischen Stränge (18) an die Deckschicht (14) und ein zweites Klebstoffmuster (32) zum Koppeln der Deckschicht (14) an das erste Laminat umfasst.

## Revendications

1. Procédé de formation d'un stratifié comprenant :
la formation indépendante d'un premier stratifié comprenant une couche de base (16) couplée à une couche élastomérique (20) ;
la formation indépendante d'un second stratifié comprenant une couche de dessus (14) couplée à une série de torons élastiques incurvés (18) par un adhésif (24a) ; et
le couplage dudit premier stratifié audit second stratifié par ultrasons.

2. Procédé selon la revendication 1, ladite couche de base (16) étant couplée à ladite couche élastomérique (20) par ultrasons.

3. Procédé selon la revendication 1, dans lequel la couche de dessus (14) est une couche non tissée et l'adhésif est appliqué à la couche de dessus.

4. Stratifié formé par le procédé selon la revendication 1, ledit stratifié comprenant une zone adhésive (34) représentant un emplacement où ladite couche de dessus (14) est couplée à ladite série de torons élastiques incurvés (18), et une zone ultrasonore (36) représentant un emplacement où ledit premier stratifié est couplé par ultrasons audit second stratifié.

5. Stratifié selon la revendication 4, ladite zone ultrasonore (36) ne contenant pas d'adhésif.

6. Stratifié selon la revendication 4, ladite zone adhésive (34) comprenant un premier motif d'adhésif (30) pour coupler les torons élastiques incurvés (18) à la couche de dessus (14) et un second motif d'adhésif (32) pour coupler la couche de dessus (14) au premier stratifié.
